# EUROPÄISCHE PATENTANMELDUNG

(11) **EP 2 301 551 A1**
(43) Veröffentlichungstag der Anmeldung: **30.03.2011**
(21) Anmeldenummer: 10010451.2
(22) Anmeldetag: 29.07.2003
(51) Int. Cl.: A61K 31/685, A61P 35/00

(54) **Anwendung von Alkylphosphocholinen in kombination mit Antitumormedikamenten**

(30) Priorität: 30.07.2002 US 399615 P
(62) Teilanmeldung aus: 03766336.6
(71) Anmelder: Aeterna Zentaris Gmbh, 60314 Frankfurt (DE)
(72) Erfinder: Engel, Jürgen, 63755 Alzenau (DE); Günther, Eckhard, 63477 Maintal (DE); Sindermann, Herbert, 63110 Rodgau (DE)
(74) Vertreter: Polypatent

(57) **Zusammenfassung**

The invention relates to the use of alkyl phosphocholines in combination with antitumor medicaments for treating benign and malignant tumor diseases in humans and mammals. The alkyl phosphocholines can be used in an inventive combination with one or a combination of several approved cytostatics. Preferred alkyl phosphocholines are represented in formula II.

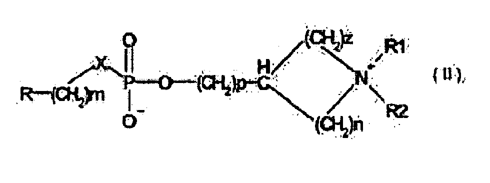

## Beschreibung

Alkylphosphocholine sind eine neue Klasse organischer Verbindungen, die vielfältige antineoplastische Aktivitäten zeigen (M. Lohmeyer und R. Bittman; Antitumor ether lipids and alkylphosphocholines, DOF, 19 (11), 1021-1037 (1994)). Die Wirkung der Alkylphosphocholine kann dabei auf verschiedenen molekularen und biochemischen Mechanismen beruhen, die zum Teil auf Ebene der Plasmamembran der Zelle stattfinden. Gut bekannt ist der Einfluß der Alkylphosphocholine auf den Inositol-Metabolismus, die Interaktion mit Phospholipasen oder die Hemmung der Protein Kinase C und damit eine generelle Beeinflussung der Signaltransduktion der Zelle durch diese Stoffklasse (K. Maly, F. Überall, C. Schubert, E. Kindler, J. Stekar, H. Brachwitz und H. H. Grunicke, Interference of new alkylphospholipid analogues with mitogenic signal transduction, Anti-Cancer Drug Design, 10, 411-425 (1995)). So zeigt das Alkylphosphocholine Perifosine wachstums-inhibitorische Eigenschaften gegenüber verschiedenen Melanom-, CNS-, Lunge-, Colon-, Prostata- und Brustkrebszelllinien mit einer IC₅₀ im Bereich von 0.2 - 20 µM (P. Hilgard, T. Klenner, J. Stekar, G. Nössner, B. Kutscher und J. Engel; D-21266, a New Heterocyclic Alkylphospholipid with Antitumor Activity, Eur. J. Cancer, 33 (3), 442-446 (1997)). Des weiteren ist bekannt, daß Perifosine Tumorzellen in der G₁-S und G₂-M Phase des Zellzyklus blockiert (V. Patel, T. Lahusen, T. Sy, E. A. Sausville, J. S. Gutkind und A. M. Senderowicz; Perifosine, a Novel Alkylphospholipid, Induces p21waf1 Expression in Squamous Carcinoma Cells through a p53-independent Pathway, Leading to Loss in Cyclin-dependent Kinase Activity and Cell Cycle Arrest, Cancer Research 62, 1401-1409 (2002)).

Es ist bekannt, daß der Einsatz von Alkylphosphocholinen vor oder zusammen mit der Strahlentherapie zu synergistischen Effekten bei der Behandlung von Tumoren führt (G.A. Ruitter, M. Verheijl, S. F. Zerp und W. J. van Blitterswijk; Alkyl-Lysophospholipids as Anticancer Agents and Enhancers of Radiation-Induced Apoptosis, Int. J. Radiation Oncology Biol. Phys., 49 (2), 415-420, 2001). Es wurde ferner berichtet, daß verschiedene Glycero-3-phospholipide, z.B. ET-18-OCH₃ in Kombination mit verschiedenen DNA-interagierenden Substanzen oder Tubulin-Bindern die Antitumor Aktivität in-vitro an verschiedenen Tumorzelllinien erhöhen (A. Noseda, M. E. Berens, J. G. White und E. J. Modest; In vitro antiproliferative activity of combinations of ether lipid analogs and DNA-Interactive agents against human tumor cells, Cancer Res., 48 (7), 1788-1791 (1988); P. Principe, H. Coulomb, C. Broquet und P. Braquet; Evaluation of combinations of antineoplastic ether phospholipids and chemotherapeutic drugs, Ant-Cancer Drugs, 3 (6), 577-587 (1992); P. Principe, H. Coulomb, J.-M. Mencia-Huerta, C. Broquet und P. Braquet; Synergistic cytotoxic effect of aza-alkylphospholipids in association with chemotherapeutic drugs, J. Lipid Mediators Cell Signalling, 10 (1-2), 171-173 (1994)).

Es konnte jetzt überraschend gezeigt werden, daß lineare Alkylphosphocholine der allgemeinen Formel I und II geeignet sind, in einer erfindungsgemäßen Kombination mit anderen Arzneimitteln zur Behandlung von gut- und bösartigen Tumorerkrankungen am Menschen und Säugetier eingesetzt zu werden. Dabei können die Verbindungen der allgemeinen Formel I und II in einer erfindungsgemäßen Kombination mit Antitumor-Substanzen eingesetzt werden. Antitumor-Substanzen können Alkylanzien, Antimetabolite, Pflanzenalkaloide, Platinverbindungen, Tumorantibiotika und Agonisten bzw. Antagonisten natürlicher Hormone sein. Die Antitumor-Substanzen können ausgewählt aber nicht darauf beschränkt sein aus: Cisplatin, Carboplatin, Oxaliplatin, Bleomycin, Doxorubicin, Methotrexat, Paclitaxel, Docetaxel, Vincristine, Vinblastine, Etoposid, Teniposid, Ifosfamid, Cyclophosphamid, 5-Fluorouracil, Fludarabin, Gemcitabin und Cytarabin.

Des weiteren können die Alkylphosphocholine der allgemeinen Formel I und II in einer beanspruchten Kombination mit Hemmstoffen der Signaltransduktion, in Form von hoch- und niedermolekularen Inhibitoren von Rezeptor- und/oder cytosolischen Kinasen eingesetzt werden. Diese Hemmstoffe können ausgewählt aber nicht darauf beschränkt sein auf Monoclonale Antikörper und heterocyclische Verbindungen.

Die der Erfindung zugrunde liegenden Alkylphosphocholine der allgemeinen Formel I und II können in Form von Fertigarzneimitteln zur Anwendung gelangen.

Die der Erfindung zugrunde liegenden Verbindungen werden durch die allgemeinen Formeln I und II beschrieben: 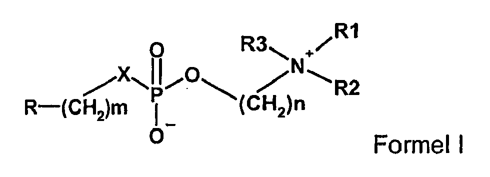 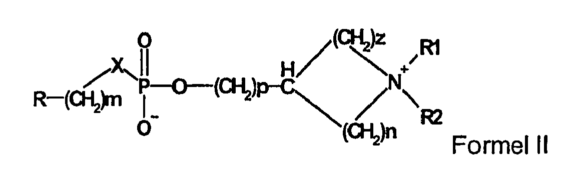 wobei unabhängig voneinander:
- n, m, p, z: eine ganze Zahl zwischen 0 und 4 bedeuten;
- X: O, S, NH;
- R: H, einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet.
- R₁, R₂, R₃: unabhängig von einander H, einen geradkettigen oder verzweigten (C₁- C₆)-Alkyl-Rest, vorzugsweise Methyl und Ethyl, einen (C₃-C₇)-Cycloalky- Rest und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet.

Gemäß einem weiteren Aspekt der Erfindung wird ein Verfahren zur Bekämpfung von Tumoren beim Menschen und in Säugetieren bereit gestellt, welches dadurch gekennzeichnet ist, daß mindestens eine der Erfindung zugrunde liegenden Verbindungen gemäß der allgemeinen Formel I und II dem Menschen oder einem Säugetier in einer für die Tumorbehandlung wirksamen Menge vor oder während einer Behandlung mit zugelassenen Antitumor-Substanzen verabreicht wird.

Die für die Behandlung zu verabreichende therapeutisch effektive Dosis der jeweiligen der Erfindung zugrunde liegenden Verbindung der allgemeinen Formel I und II richtet sich u.a. nach der Art und dem Stadium der Tumorerkrankung, dem Alter und Geschlecht des Patienten, der Art der Verabreichung und der Dauer der Behandlung.

Die der Erfindung zugrunde liegenden Verbindungen können in einem Arzneimittel als flüssige, halbfeste und feste Arzneiformen verabreicht werden. Dies erfolgt in der jeweils geeigneten Weise in Form von Aerosolen, Pulver, Puder und Streupuder, Tabletten, Dragees, Emulsionen, Schäume, Lösungen, Suspensionen, Gele, Salben, Pasten, Pillen, Pastillen, Kapseln oder Suppositorien.

### Ausführungsbeispiele:

1. Gabe von Perifosine (D-21 266) in Kombination mit Cisplatin

| | |
|---|---|
| In-vivo Versuch: | DMBA induziertes Mamma-Karzinom Modell an der Ratte |
| | |
| Versuchstier: | Sprague-Dawley Ratte, weiblich |
| | |
| Durchführung: | Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt. |
| | |
| Gabe: | Perifosin 14 x 6.81 mg/kg p.o. |
| | Cisplatin 4 x 1 mg/kg i.p. |
| | |
| Effekt: | Die Reduktion des Tumors war durch die Kombinationsbehandlung deutlich stärker und länger als durch die jeweilige Einzelbehandlung. |

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Cisplatin | 0.9 | 410 | 0.120 |
| Perifosin (D-21266) + Cisplatin | 0.8 | -75 | <0.001 |

2. Gabe von Perifosine in Kombination mit Cyclophosphamid

| | |
|---|---|
| In-vivo Versuch: | DMBA induziertes Mamma-Karzinom Modell an der Ratte |
| | |
| Versuchstier: | Sprague-Dawley Ratte, weiblich |
| | |
| Durchführung: | Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt. |
| | |
| Gabe: | Perifosin 14 x 6.81 mg/kg p.o. |
| | Cyclophosphamid 100 mg/kg, VZ 0, i.v. |
| Effekt: | Die Reduktion des Tumors war durch die Kombinationsbehandlung deutlich stärker und länger als durch die jeweilige Einzelbehandlung. |

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Cyclophosphamid | 0.9 | 500 | 0.011 |
| Perifosin (D-21266) + Cyclophosphamid | 0.8 | -83.3 | <0.001 |

3. Gabe von Perifosine in Kombination mit Adriamycin

| | |
|---|---|
| In-vivo Versuch: | DMBA induziertes Mamma-Karzinom Modell an der Ratte |
| | |
| Versuchstier: | Sprague-Dawley Ratte, weiblich |
| | |
| Durchführung: | Die Induktion des Mamma-Karzinoms erfolgte durch einmalige orale Gabe von DMBA. Die Tiere erhielten von Tag 0 bis Tag 14 Perifosin und wurden bis Tag 42 beobachtet. Das Gewicht der Tumormasse wurde mittels Palpation und Vergleich mit Plastikmodellen ermittelt. Das Ausgangsgewicht wird gleich 100% gesetzt. |
| | |
| Gabe: | Perifosin 14 x 6.81 mg/kg p.o. |
| | Adriamycin 4 x 2.15 mg/kg i.p. |
| | |
| Effekt: | Die Reduktion des Tumors war durch die Kombinationsbehandlung deutlich stärker und länger als durch die jeweilige Einzelbehandlung. |

| Behandlung | Tumor Ausgangsgewicht [g] | Tag 21 Veränderung in [%] | p Test vs. Kontrolle |
|---|---|---|---|
| Kontrolle | 1.0 | 875 | - |
| Perifosin (D-21266) | 0.9 | -25 | <0.001 |
| Adriamycin | 1.0 | 781.3 | 0.197 |
| Perifosin (D-21266) + Adriamycin | 01.0 | -70 | <0.001 |

## Patentansprüche

1. Verwendung von Alkylphosphocholinen der allgemeinen Formel I und II 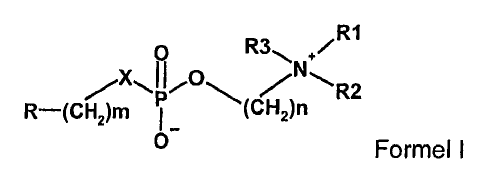 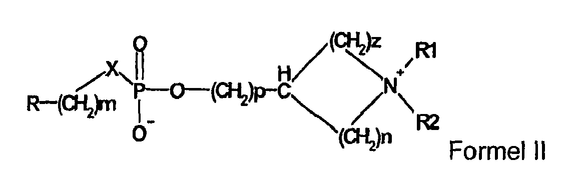 wobei unabhängig voneinander:
n, m, p, z eine ganze Zahl zwischen 0 und 4 bedeuten;
X O, S, NH bedeuten;
R H, einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet;
R₁, R₂, R₃ unabhängig von einander H, einen geradkettigen oder verzweigten (C₁- C₆)-Alkyl-Rest, vorzugsweise Methyl und Ethyl, einen (C₃-C₇)- Cycloalky-Rest und welcher unsubstituiert oder wahlweise an dem gleichen oder an verschiedenen C-Atomen mit ein, zwei oder mehreren Halogen, Nitro, Cyano, Hydroxy, (C₁-C₆)-Alkoxy, Amino, Mono-(C₁-C₄) Alkylamino oder Di- (C₁-C₄)-Alkylamino Resten substituiert sein kann, bedeutet,
zur Herstellung eines Arzneimittels zur Behandlung von gutartigen und bösartigen Tumorerkrankungen vor und/oder während der Behandlung mit einem zugelassenen Antitumormedikament.

2. Verwendung von Alkylphosphocholinen der allgemeinen Formel I gemäß Anspruch 1 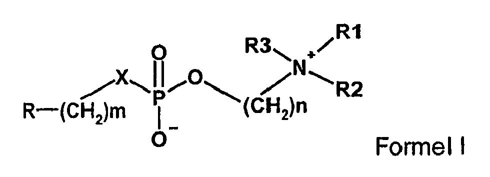 wobei unabhängig voneinander:
n die ganze Zahl 1 oder 2 bedeutet;
m die ganze Zahl 1 bedeutet;
X O bedeutet;
R H, einen geradkettigen oder verzweigten (C₁-C₁₇)-Alkyl-Rest, welcher gesättigt oder mit ein bis drei Doppel- und/oder Dreifachbindungen ungesättigt sein kann, bedeutet;
R₁, R₂, R₃ unabhängig von einander H, einen geradkettigen oder verzweigten (C₁- C₆)-Alkyl-Rest, vorzugsweise Methyl und Ethyl, einen (C₃-C₇)- Cycloalky-Rest sein kann, bedeutet,
zur Herstellung eines Arzneimittels zur Behandlung von gutartigen und bösartigen Tumorerkrankungen vor und/oder während der Behandlung mit einem zugelassenen Antitumormedikament.

3. Verwendung von Alkylphosphocholinen der allgemeinen Formel II gemäß Anspruch 1 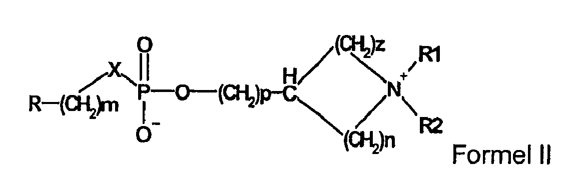 wobei unabhängig voneinander:
m, p die ganze Zahl 1 bedeutet;
n, z die ganze Zahl 2 bedeutet;
X O bedeutet;
R H, einen geradkettigen oder verzweigten (C₁-C₁₇)-Alkyl-Rest, welcher gesättigt oder mit ein bis drei Doppel- und/oder Dreifachbindungen ungesättigt sein kann, bedeutet;
R₁, R₂, R₃ unabhängig von einander H, einen geradkettigen oder verzweigten (C₁- C₆)-Alkyl-Rest, vorzugsweise Methyl und Ethyl, einen (C₃-C₇)-Cycloalky- Rest sein kann, bedeutet,
zur Herstellung eines Arzneimittels zur Behandlung von gutartigen und bösartigen Tumorerkrankungen vor und/oder während der Behandlung mit einem zugelassenen Antitumormedikament.

4. Verwendung von Octadecyl-(1,1-dimethyl-piperidinio-4-yl)-phosphat gemäß Anspruch 1 zur Herstellung eines Arzneimittels zur Behandlung von gutartigen und bösartigen Tumorerkrankungen vor und/oder während der Behandlung mit einem zugelassenen Antitumormedikament.

5. Verwendung von Alkylphosphocholinen der allgemeinen Formel I und II gemäß den Ansprüchen 1 bis 4, wobei die zugelassenen Antitumormedikamente Alkylantien, Antimetabolite, Pflanzenalkaloide, Platinverbindungen, Tumorantibiotika und Agonisten bzw. Antagonisten natürlicher Hormone sein können.

6. Verwendung nach Anspruch 5, wobei die Antitumormedikamente Cisplatin, Cyclophosphamid oder Adriamycin sein können.

7. Verwendung von Alkylphosphocholinen der allgemeinen Formel I und II gemäß den Ansprüchen 1 bis 4, wobei die zugelassenen Antitumormedikamente Hemmstoffe der Signaltransduktion in Form von hoch- und niedermolekularen Inhibitoren von Rezeptor- und/oder cytosolischen Kinasen sein können.

8. Verwendung nach Anspruch 7, wobei die Hemmstoffe monoklonale Antikörper oder heterocyclische Verbindungen sein können.

9. Verwendung von Alkylphosphocholinen der allgemeinen Formel I und II nach den Ansprüchen 1 bis 8 in einer für die Behandlung wirksamen therapeutischen Dosis vor und/oder während der Behandlung mit einem zugelassenen Antitumormedikament.

10. Verwendung von Alkylphosphocholinen der allgemeinen Formel I und II nach den Ansprüchen 1 bis 9, wobei das zugelassene Antitumormedikament eine Kombination verschiedener Zytostatika ist.

11. Verwendung von Alkylphosphocholinen der Formel I und II nach den Ansprüchen 1 bis 4, zur Herstellung eines Arzneimittels zur Behandlung von gutartigen und bösartigen Tumorerkrankungen vor und/oder während der Behandlung mit einem zugelassenen Antitumormedikament, **dadurch gekennzeichnet, dass** das Arzneimittel neben dem Alkylphosphocholin der Formel I und II die üblichen pharmazeutischen Träger, Hilfsstoffe und/oder Verdünnungsmittel enthält.

12. Arzneimittel, enthaltend mindestens ein Alkylphosphocholin der allgemeinen Formel I und II und gegebenenfalls Träger- und/oder Hilfsstoffe zur Verwendung in der Behandlung von gutartigen und bösartigen Tumorerkrankungen vor und/oder während der Behandlung mit einem zugelassenen Antitumormedikament.
